Europäisches Patentamt

**European Patent Office**

**Office européen des brevets**

(11) Publication number: **0 311 999**
**A2**

## EUROPEAN PATENT APPLICATION

(21) Application number: 88116902.3

(22) Date of filing: 12.10.88

(51) Int. Cl.⁴: **C07H 19/067 , C07H 19/167**

(30) Priority: 16.10.87 JP 262409/87

(43) Date of publication of application:
**19.04.89 Bulletin 89/16**

(84) Designated Contracting States:
**AT BE CH DE ES FR GB GR IT LI LU NL SE**

(71) Applicant: **Takeda Chemical Industries, Ltd.**
**27, Doshomachi 2-chome Higashi-ku**
**Osaka-shi Osaka, 541(JP)**

(72) Inventor: **Shibutani, Kosaku**
**506-7, Sendoh Yoneda-cho**
**Kakogawa Hyogo 675(JP)**
Inventor: **Toyota, Takeshi**
**1-3, Oharano-kamisatokatsuyamacho**
**Nishikyo-ku Kyoto 610-11(JP)**

(74) Representative: **von Kreisler, Alek,**
**Dipl.-Chem. et al**
**Patentanwälte Von Kreisler-Selting-Werner**
**Deichmannhaus am Hauptbahnhof**
**D-5000 Köln 1(DE)**

(54) **Method for producing calcium 5'-ribonucleotide.**

(57) Crystals of calcium 5'-ribonucleotide can be obtained by adjusting pH of about 0.25 to 0.63 M aqueous solution of 5'-ribonucleotide to 8.2-8.7, and to said pH-adjusted solution adding gradually with stirring an aqueous solution of a calcium ion donor of the same or more molarity while the mixture is kept at about 85-95 °C, yield and production being improved markedly.

EP 0 311 999 A2

## Method for Producing Calcium 5'-Ribonucleotide

This invention relates to the method for production of crystals of calcium 5'-ribonucleotide with high productivity and high quality.

Following procedures are known for production of crystals of calcium 5'-ribonucleotide:

(1) A procedure that during and/or after formation of a calcium salt of 5'-ribonucleotide in an aqueous solution, the solution is heated to 60°C or more, and then cooled so that the salt may be precipitated. (Japanese Examined Patent Publication No. 4493/1972)

(2) A procedure that an amorphous calcium salt of 5'-ribonucleotide is dissolved or suspended in water or an aqueous solution containing a hydrophilic solvent and kept at room temperature or an elevated temperature for a long time, so that the salt may be transformed into the crystalline calcium salt of 5'-ribonucleotide. (Japanese Examined Patent Publication No.16783/1972)

(3) A procedure characterized in that in production of the corresponding calcium salt by double decomposition of a mixture of an aqueous solution of a sodium salt of 5'-ribonucleotide and an aqueous solution or a suspension of a calcium compound, the mixture is prepared so that the pH may be in the range of 5.5 to 7.0 by previous addition of an acid to the aqueous solutions or suspension or by addition to the mixture, followed by continuous or stepwise adjustment of pH of the said mixture from the acidic region to the alkaline region (6.5-8.5) along with deposition or formation of crystals. (Japanese Examined Patent Publication No.43633/1973)

(4) A procedure that a solution containing one or more of free 5'-ribonucleotide or salts thereof and a solution of calcium hydroxide or calcium chloride are added dropwise at the same time into a crystallization tank with stirring so that crystals of corresponding calcium salts may deposit. (Japanese Patent Application Laid-Open No.78665/1984)

Salts, especially sodium salts, of tasty 5'-ribonucleotide such as 5'-inosinic acid and 5'-guanylic acid, are widely used as nucleic acid seasonings, having a problem that their tasty character is lost by decomposition by phosphatase in some kinds of good to be seasoned. On the other hand, calcium 5'-ribonucleotide has a low solubility in water. Seasoning with the calcium salt based on this property is very effective, because the salt is paractically insoluble below 50°C where phosphatase is active and becomes soluble in food to show its seasoning effect over 50°C where phosphatase is deactivated. In this case coating of the particles of calcium 5'-ribonucleotide with an edible coating agent that is melted over 50°C, such as fats and oils, is desirable for prevention of decomposition by phosphatase. Though calcium 5'-ribonucleotide is thus useful as seasonings, industrially satisfactory method for its production has not been established yet. The conventional methods have following problems in production of crystals of calcium 5'-ribonucleotide; for example, according to the method described in Japanese Examined Patent Publication No.4493/1972, reflux by heating or heating under elevated pressure is necessary for production of the desired product in a short time, and therefore pyrolysis might occur in some procedures. Stirring with heating for a long time is necessary when the reaction is carried out below 60°C, and therefore products with unsatisfactory quality might be obtained in some procedures; products might not conform to the quality standards defined by "Japanese Standards of Food Additives, the 5th edition" because of an excess of water-soluble substance in the product. Only a small amount of the product is obtained in one run because the concentration of 5'-ribonucleotide at the start of production is as low as 10% in any of the methods, and thus the methods are not industrially advantageous with such low productivity.

Taking the circumstances described above into consideration, the inventors made researches in economically effective production of crystals of calcium 5'-nucleotide with excellent quality, and completed this invention for production of crystals of calcium 5'-nucleotide with excellent quality by finding out appropriate conditions with respect to 1) concentration at the start of production, 2) pH of the solution of 5'-ribonucleotide, 3) temperature of crystallization, and, 4) molar ratio of 5'-ribonucleotide to calcium.

Namely, this invention relates to the method for producing calcium 5'-ribonucleotide, which comprises about 0.25 to 0.63 M aqueous solution of 5'-ribonucleotide is adjusted to pH about 8.2-8.7, to which an aqueous solution of a calcium ion donor of the same or more molarity is added gradually with stirring while the solution is kept at about 85-95°C, to give crystals of calcium 5'-ribonucleotide. The 5'-ribonucleotide used as the starting material in this invention includes 5'-inosinic acid, 5'-guanylic acid, 5'-uridylic acid, and 5'-cytidylic acid, and the water-soluble salts thereof, and the mixture of two or more of these compounds. The said water-soluble salts include alkali metal salts such as sodium, potassium and lithium salts, and ammonium salts. This invention is desirably applied to tasty 5'-inosinic acid and 5'-quanylic acid and water-soluble salts thereof. In this invention the aqueous solution of 5'-ribonucleotide of a relatively high concentration that is about 0.25-0.63 M is used as described above, which is one of the characteristics of this invention. The aqueous solution described above is adjusted to pH about 8.2-8.7; the agents used for this pH adjustment include sodium hydroxide, potassium hydroxide, and ammonia water, among which sodium hydroxide is most desirable. When an aqueous solution of pH 9 or higher or pH 8 or lower is used, separation of the desired crystals is less efficient. A hydrophilic organic solvent may be added to the crystallizing solution for improvement of yield. Such hydrophilic organic solvents include methanol, ethanol and acetone, each of which is used generally at the concentration of about 5-30% volume/volume. The solvent is added desirably after the crystallizing solution is cooled below the boiling point of the hydrophilic organic solvent.

The calcium ion donors used in this invention are desirably calcium compounds that are soluble in water with the solubility of about 10% or more, for example, and show pH of not more than about 9.5 after addition of the necessary amount to the reaction mixture. Usually, the calcium ion donors are used as an aqueous solution having pH 7.1 to 8.5. Such calcium compounds include calcium chloride and calcium acetate, among which calcium chloride is used most desirably.

The reaction for production of calcium 5'-ribonucleotide is performed at about 85-95° C as described above; reaction at a temperature in this range will result in efficient separation of crystals with high quality. The amount of the calcium ion donor used in this reaction is arbitrary as far as it is equimolar or more as compared with the molarity of the starting material 5'-ribonucleotide, desirably the molar ratio being generally 1-3. The concentration is desirably 10% W/V or more for a good yield. The calcium ion donor is added gradually to the aqueous solution described above with stirring so that the reaction may proceed homogeneously and calcium 5'-ribonucleotide with good crystallinity may be obtained. Usually it is desirable to add dropwise to the surface of the aqueous solution of 5'-ribonucleotide over about 3 to 7 hours so that the molar ratio of calcium ion may reach the value described above. The pH on completion of the dropwise addition is desirably not more than about 9.5. Along with the addition the desired product deposits as crystals, but better crystals are obtained usually by keeping the mixture at about 85-95° C for further about one hour after completion of the addition followed by gradual cooling to about 30° C in about 10 hours. However separation at a higher temperature may be performed if necessary.

The method for production of this invention enables production and crystallization of calcium 5'-ribonucleotide at a higher concentration as compared with the conventional methods, yield and productivity being improved markedly. This method consists in production of calcium 5'-ribonucleotide utilizing reaction-crystallization and granulation-crystallization; crystallization under the conditions of this invention improves separation of crystals and the method is suitable for industrial production. Because of the lower solubility of the crystals obtained, the crystals after coating are stable, being resistant to degradation with phosphatase, and therefore can be utilized advantageously as additives for various processed food and as starting materials for seasoning preparations.

In the following this invention is illustrated in more concrete with Experiment and Examples. Hereinafter % W/V and % W/W mean % weight/volume and % weight/weight, respectively.

Experiment 1

A mixture of sodium 5'-inosinate and sodium 5'-guanylate in about equal amounts (Ribotide®, manufactured by Takeda Chemical Industries Ltd.) was dissolved in water at the concentration of 15% W/V (0.25 mole), and the solutions of pH 7.0, 7.7, 8.2, 8.5, 8.7, and 9.0, as listed in Table 1, were prepared by addition of sodium hydroxide. Seven hundred ml each of the solutions was kept at 90° C with stirring with a paddle agitator, to which 290 ml of a solution of calcium chloride (conforming to the standards defined by "Japanese Standards of Food Additives, the 5th Edition", the same preparation is used hereinafter) of 15% W/V (pH 7.5) was added dropwise over about 5 hours, to give crystals of calcium 5'-ribotide. Then the mixture was allowed to get cool, the crystalline slurry was separated and dried in a vacuum desiccator. Separation, molar yield, content, and water-soluble substance of the crystals obtained under various conditions for crystallization were investigated.

Table 1

| pH of starting solution | molar yield(%) | separation | content (% W/W) | water-soluble substance(mg) |
|---|---|---|---|---|
| 9.0 | 97.5 | 4 | 82.1 | 144 |
| 8.7 | 98.2 | 2 | 85.0 | 60 |
| 8.5 | 99.0 | 1 | 87.9 | 41 |
| 8.2 | 98.5 | 3 | 93.9 | 39 |
| 7.7 | 98.2 | 3 | 93.4 | 46 |
| 7.0 | 98.0 | 4 | 86.8 | 47 |

| Note 1) | | | | |
|---|---|---|---|---|
| Separation was evaluated on the basis of the time required for filtration under reduced pressure (-760 mmHg) of 500 ml of the crystalline slurry through a glass filter (25G-3). separation-1: less than 10 seconds separation-2: 10 - 19 seconds separatoon-3: 20 - 60 seconds separation-4: more than 61 seconds When separation is 1 or 2, the procedure is industrially advantageous. | | | | |

| Note 2) | | | | |
|---|---|---|---|---|
| Content and water-soluble substance were determined according to the methods described in the section "Calcium 5'-ribonucleotide" in "Japanses Standards of Food Additives, the 5th Edition". The standard values for Content and Wat r-soluble substance are 97-102% W/W and not more than 80 mg, respectively. | | | | |

As shown in Table 1, separation of crystals was good when the pH of the starting solution of sodium 5'-ribonucleotide was 8.2, 8.5, and 8.7; especially when the pH was 8.5, the aggregation between crystals was the strongest so that most of the crystals were large granules and the best separation of crystals was attained. At these pH's, crystals obtained were of satisfactory quality also with respect to content and water-soluble substance. At pH 8.5, the ratio of calcium 5'-guanylate to calcium 5'-inosinate in the crystals was about 1.2:1.0, the content of calcium 5'-guanylate being higher. The equilibrium water content in the crystals was 13.5% at the temperature of 40°C and at the relative humidity (RH) of 75%.

Experiment 2

Seven hundred ml of an aqueous solution of Ribotide® which had been adjusted to pH 8.5 and to the concentration of 15% W/V was kept at 100, 95, 90, 85, 80, 70, or 55°C as shown in Table 2, to which 290 ml of a solution of calcium chloride at the concentration of 22% W/V (pH 7.5) was added dropwise at a constant rate with stirring over 5 hours, to give crystals of calcium 5'-ribonucleotide. Similarly to Experiment 1 the crystals were separated, dried, and subjected to evaluations of the same items.

Table 2

| crystallization temperature(°C) | molar yield(%) | separation | content (% W/V) | water-soluble substance(mg) |
|---|---|---|---|---|
| 100 | 90.1 | 3 | 99.3 | 60 |
| 95 | 90.1 | 1 | 99.5 | 45 |
| 90 | 90.1 | 1 | 99.6 | 44 |
| 85 | 89.5 | 2 | 99.6 | 65 |
| 80 | 86.1 | 2 | 99.3 | 97 |
| 70 | 86.0 | 3 | 99.4 | 82 |
| 55 | 86.7 | 4 | 99.9 | 98 |

As shown in Table 2, separation was good at the crystallization temperature of 85, 90, and 95°C where crystals with lower content of water-soluble substance and with good separation were obtained.

Experiment 3

Aqueous solutions of Ribotide® of the concentrations of 25, 20, 15, and 10% W/V were prepared as shown in Table 3, and pH of each solution was adjusted to 8.5 by addition of sodium hydroxide. Seven hundred ml each of the solutions was kept at 90°C, to which 290 ml of a solution of calcium chloride of 10-25% W/V (pH 7.5) was added dropwise at a constant rate over 5 hours, to give crystals of calcium 5'-ribonucleotide. Similarly to Experiment 1, the crystals were separated, dried, and subjected to evaluations of the same items.

Table 3

| concentration of Ribotide® in starting solution (% W/V) | molar yield (%) | separation | content (% W/W) | water-soluble substance(mg) |
|---|---|---|---|---|
| 30(0.75) | 95.0 | 3 | 97.0 | 70 |
| 25(0.63) | 93.5 | 2 | 98.5 | 47 |
| 20(0.50) | 91.3 | 1 | 99.3 | 38 |
| 15(0.38) | 87.9 | 1 | 99.0 | 41 |
| 10(0.25) | 85.4 | 1 | 98.6 | 45 |
| 7(0.18) | 83.5 | 2 | 98.5 | 49 |
| The values in parentheses are molarities. | | | | |

As shown in Table 3, when the concentration was 10, 15, 20, and 25%, crystals with satisfactory yield, separation, and quality were obtained.

Experiment 4

Seven hundred ml of the aqueous solution of Ribotide® which had been adjusted to pH 8.5 and to the concentration of 15% W/V was kept at 90°C, to which a solution (290 ml, pH 7.5) of calcium chloride was added dropwise at a constant rate over about 5 hours until 1.05, 1.5, 2.2, or 3.0 moles of calcium chloride was added per one mole of 5'-ribonucleotide as shown in Table 4, to give crystals of calcium 5'-ribonucleotide. Similarly to Experiment 1, the crystals were separated, dried, and subjected to evaluations of the same items.

Table 4

| moles of CaCl$_2$ | molar yield(%) | separation | content (% W/W) | water-soluble substance(mg) |
|---|---|---|---|---|
| 3.0 | 91.5 | 2 | 99.2 | 45 |
| 2.2 | 90.1 | 1 | 99.6 | 44 |
| 1.5 | 87.9 | 1 | 99.0 | 41 |
| 1.05 | 85.4 | 2 | 99.1 | 40 |

As shown in Table 4, the higher the molar ratio of calcium chloride, the higher the yield, the better the separation, and the better the quality.

Example 1

Ribotide®, 140 g, was dissolved in 600 ml of water, which was adjusted to pH 8.5 with a solution of sodium hydroxide, and diluted to the final concentration of 15% W/V. The resultant solution was kept at 90°C with stirring (a paddle agitator, 130-150 rpm), to which 290 ml of a solution of calcium chloride of 15% W/V (pH 7.5) was added dropwise at a constant rate over about 5 hours, to give crystals of calcium 5'-ribonucleotide. The pH during the reaction was in the range of 8.6-9.3. After further one hour of stirring following completion of dropwise addition, the mixture was cooled gradually to 30°C in about 10 hours. The crystals of calcium 5'-ribonucleotide thus deposited were separated by a minicentrifuge; separation was very good. The crystals obtained by separation were dried in an electric vacuum desiccator (temperature was set at 60°C) for 5 hours, to give 108 g of crystals of calcium 5'-ribonucleotide. The molar yield was 87.9%. The quality of the crystals conformed to the values specified in "Japanese Standards of Food Additives, the 5th Edition".

Example 2

Ribotide®, 30 kg, was dissolved in 120 l of water, which was adjusted to pH 8.5 with a solution of sodium hydroxide, and diluted to the final concentration of 15% W/V. The resultant solution was kept at 90°C with stirring (100 rpm), to which 62 l of a solution of calcium chloride of 15% W/V (pH 7.5) was added dropwise at a constant rate over about 5 hours, to give crystals of calcium 5'-ribonucleotide. The pH of the crystallizing slurry was 9.2. After further one hour of stirring, the mixture was cooled gradually to 30°C in about 10 hours. The crystals of calcium 5'-ribonucleotide thus deposited were separated by a 32-inch vertical bottom discharge centrifuge; separation was very good. The molar yield was 89.1%. The quality of the crystals conformed to the values specified in "Japanese Standards of Food Additives, the 5th Edition".

Example 3

Ribotide®, 1440 kg, was dissolved in 5500 l of water, which was adjusted to pH 8.5 with a solution of sodium hydroxide, and diluted to the final concentration of 15% W/V. The resultant solution was kept at 90°C with stirring (45 rpm), to which 3000 l of a solution of calcium chloride of 15% W/V (pH 7.5) was added dropwise at a constant rate over about 5 hours, to give crystals of calcium 5'-ribonucleotide. After one hour of stirring, the mixture was cooled gradually to 30°C in about 10 hours. The crystals of calcium 5'-ribonucleotide thus deposited were separated by a 36-inch vertical bottom discharge centrifuge; separation was very good. The crystals thus separated were dried in a warm-water-circulating type chamber vacuum dryer (temperature was set at 90°C) for about 30 hours, to give 1170 kg of crystals of calcium 5'-ribonucleotide. The molar yield was 90.2%. The quality of the crystals conformed to the values specified in "Japanese Standards of Food Additives, the 5th Edition".

Example 4

Sodium 5′-inosinate, 91 g, was dissolved in 600 ml of water, which was adjusted to pH 8.5 with a solution of sodium hydroxide, and diluted to the final concentration of 10% W/V. The resultant solution was kept at 90°C with stirring (130-150 rpm), to which 290 ml of a solution of calcium chloride of 10% W/V (pH 7.5) was added dropwise at a constant rate over about 5 hours, to give crystals of calcium 5′-inosinate. After one hour of stirring, the mixture was cooled gradually to 30°C in about 10 hours. The crystals of calcium 5′-inosinate thus deposited were separated by a minicentrifuge; separation was good. The crystals thus separated were dried similarly to Example 1, to give 70 g of crystals of calcium 5′-inosinate. The molar yield was 89.0%. The content of the crystals was 98.8%.

Example 5

Sodium 5′-guanylate, 91 g, was dissolved in 600 ml of water, which was adjusted to pH 8.5 with a solution of sodium hydroxide, and diluted to the final concentration of 10% W/V. The resultant solution was kept at 90°C with stirring (130-150 rpm), to which 290 ml of a solution of calcium chloride of 10% W/V (pH 7.5) was added dropwise at a constant rate over about 5 hours, to give crystals of calcium 5′-guanylate. After one hour of stirring, the mixture was cooled gradually to 30°C in about 10 hours. The crystals of calcium 5′-inosinate thus deposited were separated by a minicentrifuge; separation was good. The crystals thus separated were dried similarly to Example 1, to give 67 g of crystals of calcium 5′-guanylate. The molar yield was 90.4%. The content of the crystals was 98.9%.

**Claims**

1. A method for producing calcium 5′-ribonucleotide, which comprises (1) adjusting pH of aqueous solution containing about 0.25 to 0.63 M of 5′-ribonucleotide to about pH 8.2 to 8.7, and (2) to said pH-adjusted solution adding gradually with stirring an aqueous solution of a calcium ion donor in the amount of the same or more molarity relative to that of the employed 5′-ribonucleotide while the mixture is kept at about 85 to 95°C, to give crystals of calcium 5′-ribonucleotide.

2. The method according to claim 1, wherein said aqueous solution is prepared from 5′-inosinic acid, 5′-guanylic acid and water-soluble salts thereof.

3. The method according to claim 1, wherein the calcium ion donors are calcium compounds that are soluble in water with the solubility of about 10% or more.

4. The method according to claim 3, wherein the calcium compound is calcium chloride or calcium acetate.

5. The method according to claim 1, wherein the molarity of calcium ion donor is 1 to 3 relative to that of the employed 5′-ribonucleotide.

6. The method according to claim 1, wherein a calcium ion donor is added dropwise to the aqueous solution of 5′-ribonucleotide over about 3 to 7 hours.